(19) Europäisches Patentamt
European Patent Office
Office européen des brevets

(11) **EP 2 803 369 B1**

(12) **EUROPÄISCHE PATENTSCHRIFT**

(45) Veröffentlichungstag und Bekanntmachung des Hinweises auf die Patenterteilung:
**30.12.2015   Patentblatt 2015/53**

(51) Int Cl.:
***A61L 9/012*** *(2006.01)*

(21) Anmeldenummer: **13167589.4**

(22) Anmeldetag: **14.05.2013**

(54) **Zweiphasige Nutzugsendpunktanzeige für Duftfreisetzungssysteme**

Two-phase Expiration Indicator for Fragrance Delivery Systems

Indicateur d'Expiration en Deux Phases pour les Systèmes de Livraison de Parfum

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**19.11.2014   Patentblatt 2014/47**

(73) Patentinhaber: **Symrise AG**
**37603 Holzminden (DE)**

(72) Erfinder:
• **Mueller, Dirk**
**37586 Dassel (DE)**
• **Wiedemann, Joern**
**37603 Holzminden (DE)**
• **Rube, Jennifer**
**37671 Hoexter (DE)**

(74) Vertreter: **Copsey, Timothy Graham et al**
**Kilburn & Strode LLP**
**20 Red Lion Street**
**London WC1R 4PJ (GB)**

(56) Entgegenhaltungen:
WO-A1-2004/087225     DE-A1-102009 045 482
DE-A1-102009 058 078

**Beschreibung**

**Gebiet der Erfindung**

Die Erfindung befindet sich auf dem Gebiet der Duftstoffe und betrifft spezielle Mischungen, die Duft- und Farbstoffe enthalten, ein Verfahren zur Nutzungsendanzeige von Beduftungssystemen sowie eine Vorrichtung, die diese Mischungen enthält.

**Stand der Technik**

Zur Beduftung und Erfrischung von Räumen werden neben Versprühsystemen insbesondere Verdunstungssysteme verwendet. Diese Verdunstungssysteme bestehen in der Regel aus Trägermaterialien, sehr einfachen Behältern sowie sehr speziellen Behältern mit Hilfsmitteln, aus denen die Duftstoffe an die Raumluft abgegeben werden. Seit langer Zeit ist man bestrebt, dem Verbraucher durch ein visuelles Signal anzuzeigen, dass der Vorrat der Duftstoffe verbraucht ist.

In der US 4,128,508 löst man diese Aufgabe durch ein Indikatorsystem enthaltend einen pH-Indikator und eine langsam verdunstende Säure oder Base. Durch die Änderung des pH-Wertes der Duftstoffmischung zeigt die veränderte Farbe des pH-Indikators das Nutzungsende an. WO 03/031966 A1 beschreibt ein System enthaltend flüchtige Farben. Durch das Verdunsten der flüchtigen Farbe wird eine Farbänderung erzielt, die den Verbrauch der Duftstoffe signalisiert. Nachteilig an beiden Systemen ist, dass die Farbänderung nicht durch die Duftstoffe selbst hervorgerufen wird, sondern durch Hilfsstoffe. Es ergibt sich zur Erzielung einer verlässlichen Nutzungsendpunktanzeige die Notwendigkeit, die Duftstoffe so auszuwählen, dass diese genau so schnell verdunsten wie die Hilfsstoffe. Die Darstellung eines kreativen und komplexen Duftes wird dadurch unmöglich oder zumindest erheblich erschwert.

GB 2444702 A offenbart nicht wässrige Mischungen enthaltend Halochrome, Duftstoffe, nicht flüchtige Säuren oder Basen sowie hochpolare, schwerflüchtige Lösungsmittel.

DE 102009058078 offenbart ein Mottenschutzsäckchen enthaltend unter anderem den Duftstoff Lavandinöl Abrialis, das Lösungsmittel Dipropylenglykol und den solvatochromen Farbstoff Nilblau.

EP0309173 offenbart Nutzungsendpunktanzeigen enthaltend Lösungsmittel, Indikatorfarbe, bevorzugt ausgewählt aus der Gruppe der Xanthene, sowie Duftstoffe.

WO2009044363 offenbart Kompositionen zur Anwendung auf Keratinmaterialien, wobei die Kompositionen ein holographisches Pigment enthalten. Nachteilig an Pigmenten ist ihre Unlöslichkeit und deshalb neigen solche Kompositionen, sofern man diese beispielsweise in Flüssigkeiten mit niedriger Viskosität einsetzen würde, zum Entmischen und folglich zu einem ungleichmäßig gefärbten Produkt.

WO2011042222 offenbart Duftabgabesysteme mit Aktivierungs- und/oder Verbrauchsanzeige, enthaltend ggf. solvatochrome Farbstoffe.

WO 2004/087225 A1 offenbart eine Abgabevorrichtung zur Abgabe eines Luftmodifizierers, wie beispielsweise eines Lufterfrischers, eines Insektizids oder eines Duftneutralisierers. Die Abgabevorrichtung umfasst einen Behälter, der eine wässrige Phase, eine nicht mischbare flüssige Phase enthält sowie einen Docht.

Bekannte Endpunktanzeigen für Duftstoffsysteme besitzen oftmals wesentliche Nachteile. Zum Ersten führt die Verdunstung der Duftstoffe zur Aufkonzentration des zurückbleibenden Farbstoffs, so dass die Farbintensität der Duftstoffkomposition allmählich zunimmt. Der Verbraucher kann folglich nicht sehr gut zwischen dem Farbumschlag und der Farbintensivierung unterscheiden und den tatsächlichen Nutzungsendpunkt nicht besonders einfach erkennen.

Oft findet der Farbumschlag nur sehr allmählich über einen langen Zeitraum von einigen Wochen bis zu Monaten statt. Infolgedessen gewöhnt sich der Konsument an die täglichen minimalen Farbveränderungen und realisiert nur schwer den vollen Umfang der Farbveränderung. Selbst wenn der ursprüngliche Farbton und der Farbton des Nutzungsendes sehr weit auseinanderliegen, führt dieser Gewöhnungseffekt dazu, dass man den tatsächlichen Nutzungsendpunkt nicht besonders einfach erkennen kann.

Weiterhin verringert sich durch die Verdunstung der Duftstoffe das Volumen bzw. die Oberfläche der Farbstofflösung. Folglich steht am Ende der Nutzung oftmals nur eine winzige Fläche bzw. ein winziges Volumen des Farbstoffs zur Anzeige zur Verfügung. Auch aus diesem Grund kann man den tatsächlichen Nutzungsendpunkt nicht besonders einfach erkennen.

**Aufgabe der Erfindung**

Die Aufgabe der vorliegenden Erfindung hat daher in erster Linie darin bestanden, Duftstoffsysteme mit Nutzungsendpunktanzeige zur Verfügung zu stellen, die die eingangs geschilderten Nachteile des Stands der Technik vermeiden. Die Systeme zeichnen sich durch eine besonders leichte Erkennbarkeit des Nutzungsendes aus, insbesondere weisen sie keine allmähliche Farbintensivierung bei Verdunstung der Duftstoffe auf, verfügen über einen plötzlichen Farbumschlag bei Nutzungsende oder/und besitzen am Ende ihrer Nutzungsdauer eine ausreichend große Anzeige des Nutzungsendes. Ferner können die Duftstoffsystem dem Verbraucher anzeigen, dass eine Duftabgabe an die Umgebung stattfindet.

**Beschreibung der Erfindung**

Die Aufgabe wird gelöst durch ein Zweiphasensystem enthaltend

a. mindestens einen solvatochromen Farbstoff,

b. Wasser,

c. mindestens einen Duftstoff

d. sowie optional mindestens ein Lösungsmittel,

dadurch gekennzeichnet, dass das Zweiphasensystem aus übereinander geschichteten flüssigen Phasen besteht, wobei die Phasen nicht miteinander mischbar sind und eine gemeinsame Phasengrenzfläche besitzen.

Das Zweiphasensystem enthält bevorzugt eine untere Phase enthaltend Wasser und eine obere Phase enthaltend mindestens einen Duftstoff (Komponente c).

Die solvatochromen Farbstoffe (Komponente a) besitzen zumindest eine minimale Löslichkeit in beiden Phasen und sollten sich vorzugsweise in einer der beiden Phasen besonders stark anreichern, wobei die Konzentration des Farbstoffs in der farbstoffreichen Phase bevorzugt mindestens um das 10-fache, besonders bevorzugt um das 100-fache und ganz besonders bevorzugt um das 1000-fache höher ist als in der farbstoffarmen Phase (bestimmt mit UV/VIS-Spektrometrie bei 20°C). Es ist prinzipiell möglich, dass der Farbstoff in der oberen oder in der unteren Phase besonders stark angereichert ist, bevorzugt sind solche Zweiphasensysteme, in denen der Farbstoff besonders stark in der oberen Phase anreichert ist. Auch können die Duftstoffe entweder in der oberen oder der unteren Phase besonders stark angereichert sein, wobei die Konzentration der Duftstoffe in der duftstoffreichen Phase bevorzugt mindestens um das 10-fache, besonders bevorzugt um das 100-fache und ganz besonders bevorzugt um das 1000-fache höher ist als in der duftstoffarmen Phase. Insbesondere bevorzugt sind solche Systeme, in denen die Duftstoffe gemeinsam mit den solvatochromen Farbstoffen (Komponente a) in der oberen Phase stark angereichert sind.

Die hohe Anreicherung der solvatochromen Farbstoffe (Komponente a) in einer der beiden Phasen führt dazu, dass diese farbstoffreiche Phase sehr stark gefärbt erscheint und die jeweils andere (farbstoffarme) Phase nahezu farblos oder lediglich geringfügig gefärbt erscheint. Überraschenderweise findet beim Verdunsten der Duftstoffe anfangs kein ausgeprägter Übergang der solvatochromen Farbstoffe (Komponente a) in die farbstoffarme Phase statt. Erst bei nahezu vollständiger Verdunstung der Duftstoffe aus der farbstoffreichen Phase findet ein nennenswerter Übergang der solvatochromen Farbstoffe (Komponente a) in die ursprünglich farbstoffarme Phase statt. Die ursprünglich farbstoffarme Phase ist nun, am Nutzungsende, deutlich gefärbt, wobei der Farbumschlag plötzlich erfolgt. Der Verbraucher kann nun sehr eindeutig an der Zunahme der Farbintensität der ursprünglich farbstoffarmen Phase das Nutzungsende erkennen.

Die Farbveränderungen in der ursprünglich farbstoffreichen Phase, insbesondere die Farbveränderung im Bereich des sichtbaren Lichts und die Farbintensivierung, signalisieren dem Verbraucher, dass das System noch Duftstoffe an die Umgebung abgibt. Dieser Aspekt ist ebenfalls wichtig, um dem Verbraucher zu signalisieren, dass das Duftabgabesystem noch aktiv ist. Ferner kann sich der Verbraucher auch während der langen Nutzungszeit an einem allmählichen Farbwechsel erfreuen.

Bei dem erfindungsgemäßen Zweiphasensystem handelt es sich nicht um eine Emulsion. Emulsionen sind zwar auf mikroskopischer Ebene inhomogene Zweiphasensysteme, makroskopisch und äußerlich betrachtet erschienen diese allerdings homogen, so dass sich die erfindungsgemäßen Effekte für den Verbraucher nicht an Emulsionen zeigen.

Solvatochrome Farbstoffe (Komponente a) im Sinne der vorliegenden Erfindung sind solche Farbstoffe, bei denen der Effekt der Solvatochromie auftritt. Solvatochromie ist nach der Definition gemäß Römpp Online (http://www.roempp.com/) "eine Veränderung der Lichtabsorption einer gelösten Substanz (eines Chromophors) in Abhängigkeit vom Lösemittel". Bevorzugt einzusetzende solvatochrome Farbstoffe werden ausgewählt der Gruppe, die gebildet wird von Merocyaninen, roten Pyrazolonfarbstoffen, Azomethinfarbstoffen, Indoanilinfarbstoffen, Pyridinium-N-phenoxidbetainen, Nilrot, Reichardt-Farbstoff, Nilblau, Dimethylaminobenzaldehyd, 4-[2-N-substitertes-1,4-hydropyridin-4-ylidine)ethylidene]cyclohexa-2,5-dien-1-on, 1-(4-hydroxyphenol)-2,4,6-triphenylpyridinium, 5-Dimethylamino-5'-nitro-2,2'-bithiophen und deren Gemischen. Der besonders bevorzugte solvatochrome Farbstoff ist Nilrot.

Die solvatochromen Farbstoffe werden bevorzugt zu 0,0001 bis 0,1 Gew.-% und besonders bevorzugt zu 0,001 bis 0,01 Gew.-% in den erfindungsgemäßen Zweiphasensystemen eingesetzt, jeweils bezogen auf die Summe der Komponenten a, b, c und d.

Bevorzugt beträgt der Wasseranteil (Komponente b) in den erfindungsgemäßen Zweiphasensystemen zwischen 0,5 und 70 Gew.-%, bevorzugt 20 bis 60 Gew.-% und insbesondere bevorzugt 30- 55 Gew.-% bezogen auf die Gesamtmasse der Komponenten a, b, c und d. Der Wasseranteil ist in den erfindungsgemäßen Zweiphasensystemen insbesondere notwendig, um eine zweite Phase für die weiter oben beschriebenen erfindungsgemäßen Effekte bereitzustellen oder/und am Nutzungsende eine ausreichend große Anzeigefläche/ ein ausreichend großes Anzeigevolumen zur Verfügung zu stellen.

Duftstoffe (Komponente c) im Sinne der Erfindung sind insbesondere solche Duftstoffe, die einen angenehmen Geruchseindruck erzeugen. Bevorzugt sind Duftstoffe aus der Gruppe c1, die gebildet wird von Aldehyden, Estern, Ketalen und deren Mischungen. Besonders bevorzugt beträgt der Anteil der Gruppe c1 bezogen auf die Gesamtmasse der Komponenten c mindestens 20 Gew.-%. Die Auswahl dieser Duftstoffe trägt zu einem besonders plötzlichen Farbübergang zum Ende der Nutzung bei.

Lösungsmittel (Komponente d) sind insbesondere für Duftstoffe geeignete Lösungsmittel, bevorzugt ausgewählt aus der Gruppe der Alkohole und besonders bevorzugt aus der Gruppe, die gebildet wird von Ethanol, Propanol, Butanol, Isopropylalkohol, Propandiol, Butandiol, Pentandiol, Hexandiol, Phenylethylalkohol, 3,3 Methylmethoxybutanol, Solutol, Dipropylenglykolmonomethylether, Tripropylenglykolmonomethylether, Propylenglykolphenylether, Phenoxyethylalkohol, Propylenglycol, Dipropylenglycol, Triethylenglycol, Glycerin, 2-Methyl- 1,3-propandiol und deren Gemischen. Die Lösungsmittel werden bei Bedarf benötigt, um zumindest eine geringe Löslichkeit der solvatochromen Farbstoffe (Komponente a) in der farbstoffarmen Phase herzustellen und einen Farbübergang in die farbstoffarme Phase zum Zeitpunkt des Nutzungsendes zu gewährleisten. Außerdem lässt sich damit die anfängliche Färbung der farbstoffarmen Phase zwischen farblos und hellen Pastelltönen einstellen.

Die Zweiphasensysteme können optional Stabilisierungsmittel zur weiteren Erhöhung der (Licht-) Stabilität enthalten. Unter Stabilisierungsmitteln werden erfindungsgemäß bevorzugt Lichtschutzmittel, Antioxidantien, Konservierungsmittel und Emulgatoren verstanden.

Die erfindungsgemäßen Zweiphasensysteme können gegebenenfalls weitere Farbstoffe aus der Gruppe der nicht-solvatochromen Farbstoffe enthalten, um andere Farbnuancen oder dergleichen einzustellen.

In einer weiteren bevorzugten Form der Erfindung sind die Zweiphasensysteme frei von Emulgatoren. Man ist bestrebt, Abfüllanlagen für Flüssigkeiten mit hohen Durchsatzraten zu betreiben und arbeitet deshalb mit hohen Abfüllgeschwindigkeiten. Damit verbunden ist eine starke Durchmischung der Komponenten und es besteht die Gefahr der unerwünschten Emulsionsbildung. Durch die Abwesenheit von Emulgatoren wird eine Emulsionsbildung vermieden.

Die Erfindung betrifft auch ein Verfahren zur Nutzungsendpunktsanzeige von Duftstoffsystemen, bei dem man die erfindungsgemäßen Komponenten

    a. mindestens einen solvatochromen Farbstoff,

    b. Wasser,

    c. mindestens einen Duftstoff

    d. sowie optional mindestens ein Lösungsmittel

so in Kontakt bringt, dass ein Zweiphasensystem aus übereinander geschichteten flüssigen Phasen entsteht und dieses der Luft aussetzt, wobei ein plötzlicher Farbumschlag in dem Zweiphasensystem stattfindet, sobald die Duftstoffe nahezu vollständig an die Luft abgegeben worden sind. Bevorzugt findet in dem Zweiphasensystem ein allmählicher Farbumschlag und ein plötzlicher Farbumschlag statt.

Unter einem plötzlichen Farbumschlag wird erfindungsgemäß bevorzugt ein Farbumschlag innerhalb eine Woche, bevorzugt innerhalb von 4 Tagen und besonders bevorzugt innerhalb von einem Tag verstanden.

Unter einer nahezu vollständigen Duftstoffabgabe wird erfindungsgemäß bevorzugt eine Abgabe von mehr als 80 Gew.-% und besonders bevorzugt von mehr als 90 Gew.-% der ursprünglichen Masse der Duftstoffe an die Luft verstanden.

Der Farbumschlag findet bevorzugt zwischen den beiden Phasen statt, in der Art, dass die Farbstoffkonzentration in der ursprünglich farbstoffarmen Phase plötzlich ansteigt, nachdem mindestens 90 Gew-% der Duftstoffe an die Luft abgegeben worden sind und die Farbe der ursprünglich farbstoffarmen Phase nach dem Farbumschlag anders gefärbt ist als die farbstoffreiche Phase.

In einer weiteren bevorzugten Ausführungsform der Erfindung liegt die erfindungsgemäße Mischung in einem Behälter vor, der eine semipermeable Membran aufweist oder aus einer semipermeablen Membran besteht. Die semipermeable Membran besteht in einer bevorzugten Ausführungsform aus Polyethylen. Zumindest ein Teil des Behälters sollte lichtdurchlässig im Bereich des sichtbaren Lichts sein. Weitere Formen, in denen die erfindungsgemäßen Mischungen eingesetzt werden können sind Lufterfrischer für Räume, Geschirrspülmaschinen, Wäschetrockner, Toiletten sowie Duftöllampen mit transparentem Ölvorratsbehälter. Optional kann der Behälter einen Sichtschutz für eine der beiden Phasen enthalten, wobei die Abdeckung so erfolgen sollte, dass die Phase nicht von einem Betrachter gesehen werden kann. Bevorzugt enthält der Behälter einen Sichtschutz für die farbstoffarme Phase. In diesem Fall ist es für den Verbraucher besonders einfach, das Nutzungsende zu bestimmen.

Die Erfindung betrifft auch ein Herstellungsverfahren zur Herstellung der erfindungsgemäßen Zweiphasensysteme umfassend die Schritte:

I Bereitstellen einer Phase I enthaltend Wasser (Komponete b)

II Bereitstellen einer Phase II enthaltend Duftstoffe (Komponente c) sowie mindestens einen solvatochromen Farbstoff (Komponente a) und

III Überschichten der Phase I mit Phase II.

Die Lösungsmittel (optionale Komponente d) werden bevorzugt der Phase II im Schritt II hinzugefügt. In Schritt II wird der solvatochrome Farbstoff (Komponente a) mit den Duftstoffen und optional den Lösungsmitteln vermischt, um eine Lösung des Farbstoffs herzustellen.

Schließlich betrifft die Erfindung auch die Verwendung der erfindungsgemäßen Zweiphasensysteme zur Nutzungsendpunktanzeige von Duftstoffabgabesystemen.

**Beispiele**

Beispiel 1

Eine zweiphasige Duftstoffkomposition wurde aus folgenden Komponenten hergestellt:

|         | Einwaage in g | Komponente |
|---------|---------------|------------|
| Phase I | 25,9998       | Wasser     |
|         |               |            |
| Phase II | 59,2000      | Duftstoffe (Mischung aus Aldehyden, Estern und Ketalen) |
|         | 14,8000       | Dipropylenglycol |
|         | 0,0002        | Nilrot     |

Das Wasser wurde in ein erstes Becherglas vorgelegt. In einem zweiten Becherglas wurden die Duftstoffe, das Dipropylenglycol und das Nilrot miteinander verrührt, bis sich das Nilrot gelöst hat.

Der Inhalt des zweiten Becherglases wurde vorsichtig über die Wasserphase geschichtet. Das Becherglas wurde offen in einem Laborabzug der Luft ausgesetzt. Anfänglich ist die obere Phase (Phase II) rosa gefärbt und die untere Phase (Phase I) farblos. Mit zunehmender Verdunstung der Duftstoffe und des Dipropylenglycols nimmt die Farbintensität des rosa Farbtons allmählich zu und der Farbton verschiebt sich allmählich in die Farbrichtung Rot. Der Verdunstungsvorgang wird fortgesetzt, bis lediglich ein kleiner Ring der oberen Phase zurückbleibt. Dieser erscheint nun dunkel rot-violett. Die untere Phase bleibt sehr lange farblos und deren Volumen bleibt nahezu konstant. Erst mit dem Einsetzen der Ringbildung der oberen Phase schlägt die farblose unter Phase zunächst plötzlich in einen hell violetten Farbton um und kurz darauf plötzlich in einen tiefblauen Farbton. An der oberen Phase kann der Betrachter erkennen, dass sich der Farbton allmählich verändert und dass das System Duftstoffe abgibt. An der unteren Phase erkennt der Betrachter einen plötzlichen Farbwechsel und kann daran das Nutzungsende des Duftabgabesystems erkennen.

Beispiel 2

Es werden Portionen zu jeweils 6 g des Zweiphasensystems nach Beispiel 1 hergestellt und in klare und durchsichtige Polyethylenbecher gefüllt. Die Öffnung der Polyethylenbecher wird mit einer Polyethylenmembran verschweißt.

Die verschweißten Becher werden in ein Zimmer mit einer Temperatur von 20°C gestellt und der Massenanteil der verdunsteten Duftstoffe über die Zeit durch Wägung der verschweißten Becher bestimmt. Dabei wurde folgende Formel zu Grunde gelegt:

$$\text{Massenanteil der verdunsteten Duftstoffe} = 100 - \frac{\text{Masse zur Zeit t} * 100\%}{\text{Masse zur Zeit t=0}}$$

| Versuchszeit t in Stunden | Massenanteil verdunsteter Duftstoffe | Farbe der oberen Phase II | Farbe der unteren Phase I |
|---|---|---|---|
| 0 | 0 | hellrosa | farblos |
| 48 | 14,6 | hellrosa | farblos |
| 96 | 39,7 | rosa | farblos |
| 144 | 58,7 | rosa | farblos |
| 192 | 73,2 | dunkelrosa | farblos |
| 240 | 83,7 | roter Ring | violett |
| 384 | 93,0 | rot-violetter Ring | dunkel blau-violett |

Die Versuchsergebnisse zeigen, dass in der unteren Phase eine plötzliche Farbveränderung auftritt, nachdem der überwiegende Teil der Duftstoffe an die Umgebung abgegeben worden ist. Diese ist sehr eindeutig und kann dem Endverbraucher mit sehr großer Signalwirkung das Nutzungsende anzeigen.

Markiert man zu Beginn des Versuchs zusätzlich die Höhe der unteren Phase I in dem Becher, stellt man am Ende des Versuchs fest, dass sich diese bis zum Ende des Versuchs nicht verändert hat. Es ist folglich eine große Anzeige zur Verfügung, an der man das Nutzungsende ablesen kann.

Vergleichendes Beispiel 3

Beispiel 3 wird wiederholt, wobei in den Polyethylenbecher ausschließlich 6 g der Phase II aus Beispiel 1 gegeben werden, d.h. Phase I ist in diesem Versuch nicht vorhanden. Das zu vergleichszwecken präparierte Einphasensystem wechselt den Farbton von hellrosa, über rosa, dunkelrosa, rot und rot-violett. Wegen des allmählichen Farbübergangs zwischen sehr ähnlichen Farbtönen kann der Endverbraucher hier nur sehr ungenau das Nutzungsende erkennen. Am Ende des Versuchs verbleibt lediglich ein kleiner rot-violetter Ring am Boden des Bechers. Die größe der Anzeige ist in diesem Vergleichsfall deutlich kleiner als im erfindungsgemäßen Beispiel 2.

**Patentansprüche**

1. Zweiphasensystem enthaltend

    a. mindestens einen solvatochromen Farbstoff,
    b. Wasser,
    c. mindestens einen Duftstoff
    d. sowie optional mindestens ein Lösungsmittel,

    **dadurch gekennzeichnet, dass** das Zweiphasensystem aus übereinander geschichteten flüssigen Phasen besteht.

2. Zweiphasensystem nach Anspruch 1, **dadurch gekennzeichnet, dass** sie solvatochrome Farbstoffe (Komponente a) enthalten, die ausgewählt sind aus der Gruppe, die gebildet wird von Merocyaninen, roten Pyrazolonfarbstoffen, Azomethinfarbstoffen, Indoanilinfarbstoffen, Pyridinium-N-phenoxidbetainen, Nilrot, Reichardt-Farbstoff, Dimethylaminobenzaldehyd, 4-[2-N-substitertes-1,4-hydropyridin-4-ylidine)ethylidene]cyclohexa-2,5-dien-1-on, 1-(4-hydroxyphenol)-2,4,6-triphenylpyridinium, 5-Dimethylamino-5'-nitro-2,2'-bithiophen und deren Gemischen.

**3.** Zweiphasensystem nach Anspruch 1, **dadurch gekennzeichnet, dass** es den solvatochromen Farbstoff (Komponente a) Nilrot enthält.

**4.** Zweiphasensystem nach einem der vorhergehenden Ansprüche, **dadurch gekennzeichnet, dass** der Wasseranteil (Komponente b) zwischen 0,5 und 70% Gew.-% beträgt, bezogen auf die Gesamtmasse der Komponenten a, b, c und d.

**5.** Zweiphasensystem nach einem der vorhergehenden Ansprüche, **dadurch gekennzeichnet, dass** der Anteil der Lösungsmittel (Komponente d) zwischen 0 und und 20% Gew.-% beträgt.

**6.** Zweiphasensystem nach einem der Ansprüche 1 bis 5, **dadurch gekennzeichnet, dass** die Duftstoffe ausgewählt sind aus der Gruppe c1, die gebildet wird von Aldehyden, Estern, Ketalen und deren Mischungen.

**7.** Zweiphasensystem nach einem der Ansprüche 1 bis 6, **dadurch gekennzeichnet, dass** sie Lösungsmittel (Komponente d) enthalten, die ausgewählt sind aus der Gruppe, die gebildet wird von Ethanol, Propanol, Butanol, Isopropylalkohol, Propandiol, Butandiol, Pentandiol, Hexandiol, Phenylethylalkohol, 3,3 Methylmethoxybutanol, Solutol, Dipropylenglykolmonomethylether, Tripropylenglykolmonomethylether, Propylenglykolphenylether, Phenoxyethylalkohol, Propylenglycol, Dipropylenglycol, Triethylenglycol, Glycerin, 2-Methyl- 1,3-propandiol und deren Gemischen.

**8.** Zweiphasensystem nach einem der vorhergehenden Ansprüche, **dadurch gekennzeichnet, dass** diese frei sind von Emulgatoren.

**9.** Verfahren zur Nutzungsendpunktsanzeige von Duftstoffsystemen, bei dem man Zweiphasensysteme nach einem der vorhergehenden Ansprüche 1 bis 8 der Luft aussetzt, wobei in dem Zweiphasensystem eine Farbänderung stattfindet, sobald die Duftstoffe nahezu vollständig an die Luft abgegeben worden sind.

**10.** Verfahren nach Anspruch 11, **dadurch gekennzeichnet, dass** die Farbänderung stattfindet, nachdem mehr als 80 Gew.-% der ursprünglichen Masse der Duftstoffe an die Luft abgegeben worden ist.

**11.** Verfahren nach einem der Ansprüche 11 oder 12, **dadurch gekennzeichnet, dass** die wässrige Phase zu Beginn farblos ist.

**12.** Herstellungsverfahren zur Herstellung eines Zweiphasensystems nach einem der Ansprüche 1 bis 8 umfassend die Schritte

I. Bereitstellen einer Phase I enthaltend Wasser (Komponete b)
II. Bereitstellen einer Phase II enthaltend Duftstoffe (Komponente c) sowie mindestens einen solvatochromen Farbstoff (Komponente a) und
III. Überschichten der Phase I mit Phase II.

**13.** Lichtdurchlässiger Behälter mit einem Verschluss aus einer semipermeablen Membran oder / und einem Sichtschutz für eine der Phasen, der Zweiphasensysteme nach einem der Ansprüche 1 bis 8 enthält.

**14.** Verwendung von Zweiphasensystemen nach einem der Ansprüche 1 bis 8 zur Nutzungsendpunktanzeige von Duftstoffabgabesystemen.

## Claims

**1.** Two-phase system comprising

a. at least one solvatochromic dye,
b. water,
c. at least one fragrance
d. and optionally at least one solvent,

**characterized in that** the two-phase system consists of liquid phases layered one on top of the other.

2. Two-phase system according to Claim 1, **characterized in that** it comprises solvatochromic dyes (component a) which are selected from the group which is formed by merocyanines, red pyrazolone dyes, azomethine dyes, indoaniline dyes, pyridinium N-phenoxide betaines, Nile red, Reichardt dye, dimethylaminobenzaldehyde, 4-[2-N-substituted-1,4-hydropyridin-4-ylidene)ethylidene]cyclohexa-2,5-dien-1-one, 1-(4-hydroxy-phenol)-2,4,6-triphenylpyridinium, 5-dimethyl-amino-5'-nitro-2,2'-bithiophene and mixtures thereof.

3. Two-phase system according to Claim 1, **characterized in that** it comprises the solvatochromic dye (component a) Nile red.

4. Two-phase system according to one of the preceding claims, **characterized in that** the water fraction (component b) is between 0.5 and 70% by weight, based on the total mass of components a, b, c and d.

5. Two-phase system according to one of the preceding claims, **characterized in that** the fraction of solvents (component d) is between 0 and 20% by weight.

6. Two-phase system according to one of Claims 1 to 5, **characterized in that** the fragrances are selected from group c1 which is formed by aldehydes, esters, ketals and mixtures thereof.

7. Two-phase system according to one of Claims 1 to 6, **characterized in that** it comprises solvents (component d) which are selected from the group which is formed by ethanol, propanol, butanol, isopropyl alcohol, propanediol, butanediol, pentanediol, hexanediol, phenylethyl alcohol, 3,3-methylmethoxybutanol, Solutol, dipropylene glycol monomethyl ether, tripropylene glycol monomethyl ether, propylene glycol phenyl ether, phenoxyethyl alcohol, propylene glycol, dipropylene glycol, triethylene glycol, glycerol, 2-methyl-1,3-propanediol and mixtures thereof.

8. Two-phase system according to one of the preceding claims, **characterized in that** it is free from emulsifiers.

9. Method of indicating the expiration of fragrance systems, in which two-phase systems according to one of the preceding Claims 1 to 8 are exposed to the air, with a colour change taking place in the two-phase system as soon as the fragrances have been almost completely delivered into the air.

10. Method according to Claim 11, **characterized in that** the colour change takes place after more than 80% by weight of the original mass of the fragrances has been delivered into the air.

11. Method according to one of Claims 11 or 12, **characterized in that** the aqueous phase at the start is colourless.

12. Preparation process for producing a two-phase system according to one of Claims 1 to 8, comprising the steps

> I. provision of a phase I comprising water (component b)
> II. provision of a phase II comprising fragrances (component c) and at least one solvatochromic dye (component a) and
> III. layering phase II on top of phase I.

13. Light-permeable container with a closure made of a semipermeable membrane and/or a screen for one of the phases which comprises two-phase systems according to one of Claims 1 to 8.

14. Use of two-phase systems according to one of Claims 1 to 8 for indicating the expiration of fragrance delivery systems.

**Revendications**

1. Système biphasé, contenant

> a. au moins un colorant solvatochrome,
> b. de l'eau,
> c. au moins un parfum,
> d. et éventuellement au moins un solvant,

**caractérisé en ce que** le système biphasé est constitué de phases liquides stratifiées les unes sur les autres.

**2.** Système biphasé selon la revendication 1, **caractérisé en ce qu'**il contient des colorants solvatochromes (composant a) qui sont choisis dans le groupe constitué par les mérocyanines, les colorants pyrazolone rouges, les colorants azométhine, les colorants indoaniline, les pyridinium-N-phénoxyde-bétaïnes, le rouge de Nil, le colorant de Reichardt, le diméthylaminobenzaldéhyde, la 4-[1,4-hydropyridin-4-ylidine 2-N-substituée)éthylidène]cyclohexa-2,5-dién-1-one, le 1-(4-hydroxyphénol)-2,4,6-triphénylpyridinium, le 5-diméthylamino-5'-nitro-2,2'-bithiophène et leurs mélanges.

**3.** Système biphasé selon la revendication 1, **caractérisé en ce qu'**il contient le colorant solvatochrome (composant a) rouge de Nil.

**4.** Système biphasé selon l'une quelconque des revendications précédentes, **caractérisé en ce que** la proportion d'eau (composant b) est comprise entre 0,5 et 70 % en poids, par rapport à la masse totale des composants a, b, c et d.

**5.** Système biphasé selon l'une quelconque des revendications précédentes, **caractérisé en ce que** la proportion de solvant (composant d) est comprise entre 0 et 20 % en poids.

**6.** Système biphasé selon l'une quelconque des revendications 1 à 5, **caractérisé en ce que** les parfums sont choisis dans le groupe c1, qui est constitué par les aldéhydes, les esters, les cétals et leurs mélanges.

**7.** Système biphasé selon l'une quelconque des revendications 1 à 6, **caractérisé en ce qu'**il contient des solvants (composant d), qui sont choisis dans le groupe constitué par l'éthanol, le propanol, le butanol, l'alcool isopropylique, le propanediol, le butanediol, le pentanediol, l'hexanediol, l'alcool phényléthylique, le 3,3-méthylméthoxybutanol, le solutol, l'éther monométhylique de dipropylène glycol, l'éther monométhylique de tripropylène glycol, l'éther phénylique de propylène glycol, l'alcool phénoxyéthylique, le propylène glycol, le dipropylène glycol, le triéthylène glycol, la glycérine, le 2-méthyl-1,3-propanediol et leurs mélanges.

**8.** Système biphasé selon l'une quelconque des revendications précédentes, **caractérisé en ce qu'**il est exempt d'émulsifiants.

**9.** Procédé d'indication de l'expiration de systèmes de parfums, selon lequel des systèmes biphasés selon l'une quelconque des revendications 1 à 8 précédentes sont exposés à l'air, un changement de couleur se produisant dans le système biphasé dès que les parfums ont été émis dans l'air presque en totalité.

**10.** Procédé selon la revendication 11, **caractérisé en ce que** le changement de couleur a lieu après que plus de 80 % en poids de masse initiale des parfums ait été émise dans l'air.

**11.** Procédé selon l'une quelconque des revendications 11 ou 12, **caractérisé en ce que** la phase aqueuse est incolore au début.

**12.** Procédé de fabrication pour la fabrication d'un système biphasé selon l'une quelconque des revendications 1 à 8, comprenant les étapes suivantes :

I. la préparation d'une phase I contenant de l'eau (composant b),
II. la préparation d'une phase II contenant des parfums (composant c) et au moins un colorant solvatochrome (composant a), et
III. la stratification de la phase I avec la phase II.

**13.** Contenant translucide muni d'une fermeture en une membrane semi-perméable et/ou d'une protection visuelle pour une des phases, contenant le système biphasé selon l'une quelconque des revendications 1 à 8.

**14.** Utilisation du système biphasé selon l'une quelconque des revendications 1 à 8 pour l'indication de l'expiration de systèmes d'émission de parfums.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4128508 A
- WO 03031966 A1
- GB 2444702 A
- DE 102009058078
- EP 0309173 A
- WO 2009044363 A
- WO 2011042222 A
- WO 2004087225 A1